# EUROPEAN PATENT APPLICATION

(11) **EP 0 579 130 A2**
(43) Date of publication of application: **19.01.1994**
(21) Application number: 93111009.2
(22) Date of filing: 09.07.1993
(51) Int. Cl.: G01N 33/58, G01N 33/535, C12N 9/08

(54) **Functionalized proteins and use of same in the preparation of tracing conjugated compounds**

(30) Priority: 17.07.1992 IT FI920146
(71) Applicant: SCLAVO DIAGNOSTICI S.r.l., I-53100 Siena (IT)
(72) Inventor: Presentini, Rivo, I-53100 Siena (IT); Terrana, Benedetto, I-53034 Colle Val D'Elsa (Siena) (IT)
(74) Representative: Gervasi, Gemma, Dr.

(57) **Abstract**

Method for the obtainment of proteins functionalized by means of activated ester type groups capable of forming conjugated compounds with haptens, peptides and proteins with prolonged biochemical activity, hence utilizable as tracers.

## Description

### Field of the invention

The present invention is referred to proteins functionalized by means of activated ester type groups, further to protein treatment with an excess of homobifunctional reagents specific for amino groups. Said proteins are capable of bonding directly to heptans, peptides and proteins, through stable amidic bonds; hence, they are particularly useful in all those cases involving the utilization of such type of conjugated compounds.

### State of the art

Proteins conjugated with heptans, peptides and other proteins in general, in particular with immunoglobulins, are widely used in many sectors of biochemistry such as in the immunodiagnostic field and in the preparation of genetic probes, biosensors, and immunotoxins.

Several conjugation techniques have been developed with a view to increasing the efficiency of same and the preservation of the components biological activity (see, for example, Tijssen P. (1985) "Practice and Theory of Enzyme Immunoassays" in: Burdon R.H. and van Knippenberg P.H. (Eds): "Laboratory Techniques in Biochemistry and Molecular Biology", Elsevier, Amsterdam).

The most common conjugation techniques are based on the direct addition of homobifunctional cross-linking agents, such as glutaraldehyde and dihydroxysuccinimide esters of diacids, to the solution containing enzyme and protein (one-step process). In case of proteins with polysaccharide function, conjugated compounds are prepared wherein said polysaccharide moiety is activated with limited oxidation of aldehydes vicinal alcohols. Oxidation may be obtained by the use of meta-periodate or heterobifunctional reagents, such as maleoimide thiol. In the latter case the reaction involves the activation of a protein by means of a reagent introducing maleoimide groups, and of the other protein by means of thiol groups, followed by interaction of the two functional groups to form the conjugated compound by thioether type bonds.

Several are the disadvantages of the above conjugated compounds: those obtained by the addition of glutaraldehyde or by oxidation of the protein polysaccharide moiety tend to polymerize, mostly yielding aggregates that decrease the system efficiency by a drastic loss of the protein biological activity.

On the other hand, should heterobifunctional reagents be used, as in the classical case of maleoimidate peroxidase, labelling of entire molecules, e.g. immunoglobulins, would require their treatment with a quantity of thiolating reagents suitable for modifying the protein in a larger number of sites than actually utilized for the conjugation with the enzyme. It follows that the protein biochemical activity is reduced more than strictly required.

### Detailed description of the invention

The present invention obviates the aforesaid inconveniences thanks to a protein activated with homobifunctional reagents, such as N-hydroxysuccinimide diesters of diacids (insoluble in aqueous buffers) or N-hydroxysulphosuccinimide diesters of the same diacids (soluble in aqueous buffers).

Said reagents are capable of initiating two reciprocally competitive reactions: i.e. they can react with amino groups to form stable amidic bonds, liberating one molecule of N-hydroxysuccinimide or, respectively, N-hydroxysulphosuccinimide per reacting ester molecule; moreover, they can react with the hydroxyl ions of water to give the free acid again.

The nature of the aforementioned ester groups, i.e. N-hydroxysuccinimide and N-hydroxysulphosuccinimide, is of critical importance in the invention. Instead, of lesser importance is the nature of the diacid acting as a spacer between the two ester groups bonded to the respective amino groups. In any case, the preferred diacids have C₂₋₈ carbon atoms in the chain, such as for example succinic, tartaric, adipic, suberic, and sebacic acids.

Therefore, among the aforesaid homobifunctional reagents, particularly preferred are the N-hydroxysuccinimide diesters and N-hydroxysulphosuccinimide diesters of succinic, tartaric, adipic, suberic, and sebacic acids.

The present invention contemplates all proteins - either natural or induced - whose aminic groups are not involved in the biological activity, i.e. the proteins naturally containing aminic groups not involved in the biological activity or the proteins in which said groups, if any, have been temporarily produced, e.g., by treating the protein with reagents, such as 2,3-dimethylmaleic anhydride.

The present invention is particularly referred to peroxidase activated as mentioned above and conjugated with immunoglobulins.

According to the present invention, activated peroxidase is obtained as follows: peroxidase, preferably in a buffer, e.g. phosphate buffer, pH 7-9, at a concentration of 5-30 mg/ml, is treated with a controlled excess, e.g. 3-10 molar excesses, of a suitable homobifunctional reagent, and allowed to react at room temperature for 5-15 minutes.

Considering that peroxidase contains 7 amino groups, that only 1 or 2 of them are on the molecule surface (and, therefore, available for reacting with suitable reagents) and that their modification does not decrease the enzymatic activity, the process described above yields a large amount of enzyme, saturated by the reagent, without causing its polymerization. The reagent bound to the other side of the spacing chain keeps the second molecule of active ester unaltered, therefore, available for subsequent reactions. Hence, the enzyme is activated and can directly react with an amino group of the protein or hapten or peptide to form the desired conjugated compound.

The activated enzyme is purified from excess reagent by means of procedures suitable for maintaining the enzymatic activity of same and the activity of the active diester molecules on the enzyme surface. For example, a convenient procedure may consist in the steps of precipitating the activated enzyme with organic solvents, e.g. ethanol, methanol, acetone and the like, treating the solution with an excess of said solvent, e.g. 10 volumes, and collecting the activated enzyme by centrifuging.

If required, the product can be repeatedly washed with the precipitation solvent to obtain complete removal of free reagent and further reduction in the formation, if any, of polymeric aggregates.

Final conjugation takes place by direct dissolution of the enzyme pellet in a solution containing the protein or peptide or hapten to be labelled, e.g. at a concentration of 3-6 mg/ml, in physiological PBS type buffer, pH 7.2, by stirring to complete dissolution and allowing to react at room temperature for 2-3 hours at least. A preferred use consists of 10 moles of enzyme approx. per mole of product to be labelled (e.g. proteins as immunoglobulins). The conjugated compound can be adequately purified by standard techniques, such as Spectra/Gel AcA44 silica gel column chromatography, with collection of the fractions that correspond to the column void volume (Vo). According to this technique, the protein to be labelled remains in the physiological buffer and the modification brought about by conjugation essentially concerns only one amino group per molecule of conjugated enzyme. The biological activity of the conjugated compounds obtained is at least 50% of the initial value of the conjugated moiety.

Furthermore, by operating as described above, labelling involves 80-90% approx. of the molecules to be labelled, with a degree of substitution of 2-3 moles of enzyme per mole of product to be labelled. This is an optimal condition for using the conjugated compound in immuno enzymatic tests.

The stability of the conjugated compounds thus obtained is extremely high: consequently, they can be stored for a long time at temperature above 0°C even in dilute solution.

To conclude the claimed conjugated compounds are particularly fit for use as tracers in commercial diagnostic kits.

### EXAMPLE 1

Thirty mg of peroxidase (25 mg/ml) in 0.1 M sodium phosphate buffer, pH 8, was cooled in ice bath and caused to react with bis(N-hydroxysulphosuccinimidyl)suberate (2.1 mg), either solid or dissolved in a minimum quantity of DMSO, at room temperature for 10 minutes in the dark. The resulting product was added with cold ethanol (10 ml), agitated by vortex mixing, and centrifuged at 3000 r.p.m. for 5 min. The supernatant was removed.

The obtained precipitate was added with 10 mg of IgG (5 mg/ml) in PBS, pH 7.2, and stirred to complete pellet dissolution. The mixture was allowed to react at room temperature for 2-3 hours under slight shaking.

The sample was purified by Spectra/Gel AcA44 column equilibrated in PBS buffer. Fractions were collected corresponding to the void volume (Vo) of the column and with a ratio between optical densities at 403 nm and, respectively, at 280 nm of 0.5 min., which is equivalent to a substitution of 1.3 moles peroxidase per mole immunoglobulin.

### EXAMPLE 2

Thirty mg of peroxidase (25 mg/ml) in 0.1 M sodium phosphate buffer, pH 7.1, were cooled in ice bath and caused to react with 1.2 ml of bis(N-hydroxysulsuccinimidyl)adipate (2.15 mg/ml) in DMSO. The mixture was allowed to react at room temperature for 15 minutes in the dark. Further steps as per Example 1.

## Claims

1. Proteins functionalized by means of activated ester type groups, further to protein treatment with homobifunctional reagents specific for amino groups.

2. The proteins functionalized according to claim 1, either natural or induced, whose amino groups are not involved in the protein biological activity.

3. The proteins functionalized according to claim 2, wherein the homobifunctional reagents are N-hydroxysuccinimide or N-hydroxysulphosuccinimide diesters of C₂₋₈ diacids.

4. The proteins functionalized according to claim 3, wherein the homobifunctional reagents are: N-hydroxysuccinimide and N-hydroxysulphosuccinimide diesters of succinic, tartaric, adipic, suberic, sebacic acids.

5. The proteins functionalized according to claim 4 wherein the functionalized protein is perodixase.

6. Method for the preparation of the functionalized perodixase according to claim 5, wherein peroxidase is caused to react with an excess buffered reagent at pH 7-9.

7. The method according to claim 6, wherein the reagent excess is of 3-10 moles over peroxidase.

8. Compounds consisting of a protein according to claims 1 to 4 conjugated with proteins, haptens, peptides.

9. The conjugated compounds according to claim 8 consisting of functionalized peroxidase and immunoglobulin.

10. Method for the preparation of the conjugated compounds according to claim 9, wherein a functionalized peroxidase according to claim 5 is added to the molecule to be conjugated in PBS and allowed to react at room temperature for 2-3 hours.

11. Use of the conjugated compounds according to claim 8 for diagnostic tests.

12. The use according to claim 11, wherein the conjugated compound consists of the functionalized peroxidase according to claim 5 and immunoglobulin.
